# EUROPEAN PATENT SPECIFICATION

(11) **EP 2 914 264 B1**
(45) Date of publication and mention of the grant of the patent: **09.08.2017**
(21) Application number: 13776720.8
(22) Date of filing: 04.10.2013
(51) Int. Cl.: A61K 31/506, A61P 35/00

(54) **A 6-OXO-1,6-DIHYDRO-PYRIDAZINE DERIVATIVE FOR THE USE FOR THE TREATMENT OF HEPATOCELLULAR CARCINOMA (HCC)**
6-OXO-1,6-DIHYDRO-PYRIDAZIN-DERIVAT ZUR VERWENDUNG BEI DER BEHANDLUNG VON HEPATOZELLULÄREM KARZINOM (HCC)
DÉRIVÉ DE 6-OXO-1,6-DIHYDRO-PYRIDAZINE DESTINÉ À ÊTRE UTILISÉ DANS LE TRAITEMENT DU CARCINOME HÉPATOCELLULAIRE (CHC)

(30) Priority: 02.11.2012 EP 12007494
(43) Date of publication of application: 09.09.2015
(73) Proprietor: Merck Patent GmbH, 64293 Darmstadt (DE)
(72) Inventor: FRIESE-HAMIM, Manja, 64546 Moerfelden-Walldorf (DE); BLADT, Friedhelm, 69120 Heidelberg (DE)
(86) International application number: PCT/EP2013/002998
(87) International publication number: WO 2014/067610

(56) References cited:
- WO-A1-2010/078897

## Description

### FIELD OF THE INVENTION

This invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof for the use for the treatment of hepatocellular carcinoma (HCC).

### BACKGROUND OF THE INVENTION

The invention had the object of finding novel pharmaceutical compositions having valuable properties, in particular those which can be used for the preparation of medicaments.

Moreover, aim of this invention are new compositions for the prevention and treatment of hepatocellular carcinoma.

It has been found that 3-(1-{3-[5-(1-methyl-piperidin-4-ylmethoxy)-pyrimidin-2-yl]-benzyl}-6-oxo-1,6-dihydro-pyridazin-3-yl)-benzonitrile according to the invention or a pharmaceutically acceptable salt and/or solvate thereof has very valuable pharmacological properties while being well tolerated.

HCC is the 5th most common malignancy worldwide, with 667,000 new cases worldwide and 17,500 in USA. 80% of patients present with advanced or unresectable disease at diagnosis. In Western countries, approx. 40% of patients are eligible for a potential curative treatment (resection, transplantation, local ablation) whereas approx. 20% are eligible for chemoembolization. In well-selected patients resection and transplantation provide 5-year survival rates of 70%, 50% of patients relapse within 3 years. Here we demonstrate that 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof is active in HCC tumors with signs of active c-Met signaling.

Before the approval of sorafenib there was no effective systemic treatment increasing survival in HCC: conventional cytotoxic agents given as monotherapy or in combination regimen had low response rates and did not lead to survival advantage (Thomas MB, O'Beirne JP, Furuse J, Chan AT, Abou-Alfa G, Johnson P; Ann Surg Oncol. 2008 Apr;15(4):1008-14). However, although PFS times have been improved by sorafenib, PFS and overall survival remain limited. Secondary resistance occurs after several weeks of drug exposure. After progression there is currently no other therapeutic option. Due to the high unmet medical need in HCC alternative effective treatment options are needed.

### PRIOR ART

3-(1-{3-[5-(1-Methyl-piperidin-4-ylmethoxy)-pyrimidin-2-yl]-benzyl}-6-oxo-1,6-dihydro-pyridazin-3-yl)-benzonitrile has been described in WO 2009/006959 A1.
3-(1-{3-[5-(1-Methyl-piperidin-4-ylmethoxy)-pyrimidin-2-yl]-benzyl}-6-oxo-1,6-dihydro-pyridazin-3-yl)-benzonitrile hydrochloride hydrate has been described in WO 2009/007074 A1.

### SUMMARY OF THE INVENTION

The invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof for the use for the treatment of hepatocellular carcinoma (HCC).

Moreover, the invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate for the use for the treatment of hepatocellular carcinoma (HCC).
Moreover, the invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof, wherein the compound is administered to a patient in an amount of 100 mg to 800 mg per day.
Moreover, the invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof, wherein the compound is administered orally.
Moreover, the invention relates to the use of 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof for the manufacture of a medicament for the treatment of hepatocellular carcinoma (HCC).

The therapy with 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof or
3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate may include optionally further treatment with radiation.

The invention also relates to the solvates of the salts of the compound for use e.g. the mono- or dihydrate of the hydrochloride.

The term solvates of the compound is taken to mean adductions of inert solvent molecules onto the compounds which form owing to their mutual attractive force. Solvates are, for example, mono- or dihydrates or alcoholates. The expression "effective amount" denotes the amount of a medicament or of a pharmaceutical active ingredient which causes in a tissue, system, animal or human a biological or medical response which is sought or desired, for example, by a researcher or physician.
In addition, the expression "therapeutically effective amount" denotes an amount which, compared with a corresponding subject who has not received this amount, has the following consequence:
improved treatment, healing, prevention or elimination of a disease, syndrome, condition, complaint, disorder or side-effects or also the reduction in the advance of a disease, complaint or disorder.
The expression "therapeutically effective amount" also encompasses the amounts which are effective for increasing normal physiological function.

### Pharmaceutical salts and other forms

The said compounds according to the invention can be used in their final non-salt form. On the other hand, the present invention also encompasses these compounds for use in the form of their pharmaceutically acceptable salts, which can be derived from various organic and inorganic acids and bases by procedures known in the art. Pharmaceutically acceptable salt forms of 3-(1-{3-[5-(1-methyl-piperidin-4-ylmethoxy)-pyrimidin-2-yl]-benzyl}-6-oxo-1,6-dihydro-pyridazin-3-yl)-benzonitrile and N-((S)-2,3-dihydroxy-propyl)-3-(2-fluoro-4-iodo-phenylamino)-isonicotinamide are for the most part prepared by conventional methods.
If a compound contains a carboxyl group, one of its suitable salts can be formed by reacting the compound with a suitable base to give the corresponding base-addition salt. Such bases are, for example, alkali metal hydroxides, including potassium hydroxide, sodium hydroxide and lithium hydroxide; alkaline earth metal hydroxides, such as barium hydroxide and calcium hydroxide; alkali metal alkoxides, for example potassium ethoxide and sodium propoxide; and various organic bases, such as piperidine, diethanolamine and N-methylglutamine. The aluminium salts of the compounds are likewise included. In the case of certain compounds acid-addition salts can be formed by treating these compounds with pharmaceutically acceptable organic and inorganic acids, for example hydrogen halides, such as hydrogen chloride, hydrogen bromide or hydrogen iodide, other mineral acids and corresponding salts thereof, such as sulfate, nitrate or phosphate and the like, and alkyl- and monoarylsulfonates, such as ethanesulfonate, toluenesulfonate and benzenesulfonate, and other organic acids and corresponding salts thereof, such as acetate, trifluoroacetate, tartrate, maleate, succinate, citrate, benzoate, salicylate, ascorbate and the like. Accordingly, pharmaceutically acceptable acid-addition salts of the compounds include the following: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, palmoate, pectinate, persulfate, phenylacetate, 3-phenylpropionate, phosphate, phosphonate, phthalate, but this does not represent a restriction.

Furthermore, the base salts of the compounds according to the invention include aluminium, ammonium, calcium, copper, iron(III), iron(II), lithium, magnesium, manganese(III), manganese(II), potassium, sodium and zinc salts, but this is not intended to represent a restriction. Of the above-mentioned salts, preference is given to ammonium; the alkali metal salts sodium and potassium, and the alkaline earth metal salts calcium and magnesium. Salts of the compounds which are derived from pharmaceutically acceptable organic non-toxic bases include salts of primary, secondary and tertiary amines, substituted amines, also including naturally occurring substituted amines, cyclic amines, and basic ion exchanger resins, for example arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine and tris(hydroxymethyl)methylamine (tromethamine), but this is not intended to represent a restriction.

Compounds of the present invention which contain basic nitrogen-containing groups can be quaternised using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

The above-mentioned pharmaceutical salts which are preferred include acetate, trifluoroacetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate and tromethamine, but this is not intended to represent a restriction.

Particular preference is given to hydrochloride, dihydrochloride, hydrobromide, maleate, mesylate, phosphate, sulfate and succinate.

The acid-addition salts of basic compounds are prepared by bringing the free base form into contact with a sufficient amount of the desired acid, causing the formation of the salt in a conventional manner. The free base can be regenerated by bringing the salt form into contact with a base and isolating the free base in a conventional manner. The free base forms differ in a certain respect from the corresponding salt forms thereof with respect to certain physical properties, such as solubility in polar solvents; for the purposes of the invention, however, the salts otherwise correspond to the respective free base forms thereof.

As mentioned, the pharmaceutically acceptable base-addition salts of the compounds are formed with metals or amines, such as alkali metals and alkaline earth metals or organic amines. Preferred metals are sodium, potassium, magnesium and calcium. Preferred organic amines are N,N'-dibenzylethylenediamine, chloroprocaine, choline, diethanolamine, ethylenediamine, N-methyl-D-glucamine and procaine.

The base-addition salts of acidic compounds according to the invention are prepared by bringing the free acid form into contact with a sufficient amount of the desired base, causing the formation of the salt in a conventional manner. The free acid can be regenerated by bringing the salt form into contact with an acid and isolating the free acid in a conventional manner. The free acid forms differ in a certain respect from the corresponding salt forms thereof with respect to certain physical properties, such as solubility in polar solvents; for the purposes of the invention, however, the salts otherwise correspond to the respective free acid forms thereof.

With regard to that stated above, it can be seen that the expression "pharmaceutically acceptable salt" in the present connection is taken to mean an active ingredient which comprises a compound in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.

Pharmaceutical formulations can be administered in the form of dosage units which comprise a predetermined amount of active ingredient per dosage unit. Such a unit can comprise, for example, 0.5 mg to 1 g, preferably 1 mg to 700 mg, particularly preferably 5 mg to 100 mg, of a compound according to the invention, depending on the condition treated, the method of administration and the age, weight and condition of the patient, or pharmaceutical formulations can be administered in the form of dosage units which comprise a predetermined amount of active ingredient per dosage unit. Preferred dosage unit formulations are those which comprise a daily dose or part-dose, as indicated above, or a corresponding fraction thereof of an active ingredient. Furthermore, pharmaceutical formulations of this type can be prepared using a process which is generally known in the pharmaceutical art.

Pharmaceutical formulations can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such formulations can be prepared using all processes known in the pharmaceutical art by, for example, combining the active ingredient with the excipient(s) or adjuvant(s).

Pharmaceutical formulations adapted for oral administration can be administered as separate units, such as, for example, capsules or tablets; powders or granules; solutions or suspensions in aqueous or non-aqueous liquids; edible foams or foam foods; or oil-in-water liquid emulsions or water-in-oil liquid emulsions.

Thus, for example, in the case of oral administration in the form of a tablet or capsule, the active-ingredient component can be combined with an oral, non-toxic and pharmaceutically acceptable inert excipient, such as, for example, ethanol, glycerol, water and the like. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules are produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbant, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tabletting machine, giving lumps of non-uniform shape, which are broken up to form granules. The granules can be lubricated by addition of stearic acid, a stearate salt, talc or mineral oil in order to prevent sticking to the tablet casting moulds. The lubricated mixture is then pressed to give tablets. The compounds according to the invention can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Oral liquids, such as, for example, solution, syrups and elixirs, can be prepared in the form of dosage units so that a given quantity comprises a pre-specified amount of the compound. Syrups can be prepared by dissolving the compound in an aqueous solution with a suitable flavour, while elixirs are prepared using a non-toxic alcoholic vehicle. Suspensions can be formulated by dispersion of the compound in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners and the like, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax and the like.

The compounds and salts, solvates, tautomers and stereoisomers thereof can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds and the salts, solvates, tautomers and stereoisomers thereof can also be delivered using monoclonal antibodies as individual carriers to which the compound molecules are coupled. The compounds can also be coupled to soluble polymers as targeted medicament carriers. Such polymers may encompass polyvinylpyrrolidone, pyran copolymer, polyhydroxypropylmethacrylamidophenol, polyhydroxyethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, poly-orthoesters, polyacetals, polydihydroxypyrans, polycyanoacrylates and crosslinked or amphipathic block copolymers of hydrogels.

Pharmaceutical formulations adapted for transdermal administration can be administered as independent plasters for extended, close contact with the epidermis of the recipient. Thus, for example, the active ingredient can be delivered from the plaster by iontophoresis, as described in general terms in Pharmaceutical Research, 3(6), 318 (1986).

Pharmaceutical compounds adapted for topical administration can be formulated as ointments, creams, suspensions, lotions, powders, solutions, pastes, gels, sprays, aerosols or oils.

Pharmaceutical formulations adapted for rectal administration can be administered in the form of suppositories or enemas.

Pharmaceutical formulations adapted for nasal administration in which the carrier substance is a solid comprise a coarse powder having a particle size, for example, in the range 20-500 microns, which is administered in the manner in which snuff is taken, i.e. by rapid inhalation via the nasal passages from a container containing the powder held close to the nose. Suitable formulations for administration as nasal spray or nose drops with a liquid as carrier substance encompass active-ingredient solutions in water or oil.

Pharmaceutical formulations adapted for administration by inhalation encompass finely particulate dusts or mists, which can be generated by various types of pressurised dispensers with aerosols, nebulisers or insufflators.

Pharmaceutical formulations adapted for parenteral administration include aqueous and non-aqueous sterile injection solutions comprising antioxidants, buffers, bacteriostatics and solutes, by means of which the formulation is rendered isotonic with the blood of the recipient to be treated; and aqueous and non-aqueous sterile suspensions, which may comprise suspension media and thickeners. The formulations can be administered in single-dose or multidose containers, for example sealed ampoules and vials, and stored in freeze-dried (lyophilised) state, so that only the addition of the sterile carrier liquid, for example water for injection purposes, immediately before use is necessary. Injection solutions and suspensions prepared in accordance with the recipe can be prepared from sterile powders, granules and tablets.

It goes without saying that, in addition to the above particularly mentioned constituents, the formulations may also comprise other agents usual in the art with respect to the particular type of formulation; thus, for example, formulations which are suitable for oral administration may comprise flavours.

A therapeutically effective amount of a compound depends on a number of factors, including, for example, the age and weight of the animal, the precise condition that requires treatment, and its severity, the nature of the formulation and the method of administration, and is ultimately determined by the treating doctor or vet. However, an effective amount of a compound according to the invention is generally in the range from 0.1 to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as a single dose per day or usually in a series of part-doses (such as, for example, two, three, four, five or six) per day, so that the total daily dose is the same. An effective amount of a salt, solvate, tautomer and stereoisomer thereof can be determined as the fraction of the effective amount of the compound according to the invention *per se.* It can be assumed that similar doses are suitable for the treatment of other conditions mentioned above.

The anti-cancer treatment defined herein may be applied as a sole therapy or may involve, in addition to the composition of the invention, conventional surgery or radiotherapy.

"Treating" as used herein, means an alleviation, in whole or in part, of symptoms associated with a disorder or disease, or slowing, or halting of further progression or worsening of those symptoms, or prevention or prophylaxis of the disease or disorder in a subject at risk for developing the disease or disorder.

The term "effective amount" in connection with a compound can mean an amount capable of alleviating, in whole or in part, symptoms associated with a disorder or disease, or slowing or halting further progression or worsening of those symptoms, or preventing or providing prophylaxis for the disease or disorder in a subject having or at risk for developing a disease disclosed herein, such as cancer,

The term "therapeutically effective" or "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

### USE

3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate is suitable as pharmaceutical active ingredient for mammals, especially for humans, in the treatment of hepatocellular carcinoma.

### Experimental

### Evaluation of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate in the HCC xenograft model MHCC97H

Summary: 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate displays a greater activity than sorafenib in a hepatocarcinoma xenograft model and in HCC primary explants, all of which are characterized by high c-Met and/or HGF expression. While sorafenib led to substantial body weight loss in most HCC explant models (8/9) at all doses tested (50 mg/kg/5 out of 7 days and 60 mg/kg/qd), 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate was well tolerated in all mice as indicated by the lack of substantial weight loss of animals.

Data from pre-clinical in-house studies highlight the role of c-Met in maintenance and progression of HCC and indicate that c-Met inhibition might be an attractive treatment option for HCC. The MHCC97H cell line has been established from subcutaneous xenograft of a metastatic model of human HCC in nude mice (LCI-D20) and has a tendency to metastasize to the lungs (Wu FS, Zheng SS, Wu LJ, Teng LS, Ma ZM, Zhao WH, Wu W; Liver Int. 2007 Jun; 27(5):700-7).

MHCC97H cells co-express c-Met and HGF and also secrete alpha feto-protein (AFP), a fetal-specific glycoprotein antigen that is used as a tumor marker in the management of patients with HCC.

Treatment of established fast growing subcutaneous MHCC97H tumors with 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate completely inhibited growth and induced regressions. In comparison to 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate treatment of subcutaneous MHCC97H xenografts, administration of sorafenib resulted only in marginal anti tumor activity with no tumor regressions. To evaluate the effect of c-Met inhibition under more physiological conditions, MHCC97H cells were engrafted orthotopically in the liver of mice.

Oral administration of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate starting one week after tumor fragment implantation exhibited significant anti-tumor activity, resulting in complete regression in all mice at the end of treatment at day 35. As a surrogate endpoint, body weight was followed throughout the study. Body weight loss in the vehicle group could be observed from day 18 onward, probably caused by increased tumor burden in the liver and/or lung metastasis. In contrast, for mice treated with the c-Met inhibitor, no body weight loss could be detected. At the end of the treatment period AFP levels in the circulation were analyzed. Whereas in the control group high AFP levels were detectable, no AFP was measurable in the mice treated with 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate.

Subcutaneous MHCC97H tumor model - comparison 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate and sorafenib monotherapy:
Method: Male BalB/c nude mice (6-8 week old) where subcutaneously injected with human MHCC97H liver tumor cells and were divided into treatment groups (ten animals in one group) after the tumors were established (ca. 500mm³). Respective groups were administered orally with the 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate at different doses (10, 30 and 100 mg/kg) for 5 days on and 2 days on or daily with sorafenib (50mg/kg). At the end of treatment T/C values were calculated and tumor regrowth was observed.
Results: All doses of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate showed significant anti-tumor activity inducing tumor regression with T/C ratios of -57%, -93% and -93%, respectively and a tumor growth delay (TGD, time to reach a tumor volume of 1000mm³) of 24, 53 and more as 53 days, respectively. Treatment of sorafenib showed less anti-tumor activity compared to MSC2156119J with a T/C value of 27%.

Orthotopic MHCC97H tumor model - 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate monotherapy:
Method: In male BalB/c nude mice (7-8 week old) MHCC97H tumor fragments (2-3 mm³) where orthotopically implanted into the left lobe of the liver. After 1 week of intrahepatic implantation animals were divided into treatment groups (ten animals in one group). Respective groups were administered orally with 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate at 100 mg/kg/5 days on and 2 days off for 5 weeks. At the end of treatment, tumor size and tumor weight were measured, plasma AFP levels and lung metastases analyzed.
Results: Treatment with 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate induced significant anti-tumor activity resulting in primary tumor regression (p<0.001) and reduction lung metastases (p<0.01). AFP levels in plasma of mice analyzed at the end of treatment were also significantly reduced (p<0.001). Treatment of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate was well tolerated.

### Evaluation of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate in primary HCC explants:

Summary: To study the therapeutic potential of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate in HCC patients, the activity of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate was evaluated in a preclinical phase II type trial (PP2T trial) with human primary HCC explants. Treatment of nine subcutaneous, established primary explants resulted in 1/9 complete responses (CR), 2/9 stable diseases (SD) and marginal activity in one additional model. The activity of 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate correlated positive with the activation status of the c-Met receptor expressed in these models, as indicated by c-Met and HGF expression levels. None of the models with no or only low signs of detectable c-Met signaling (c-Met, phospho c-Met and/HGF levels) responded to 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate in monotherapy and no enhanced activity in combination with sorafenib has been observed.
Method: Male BalB/c nude mice (6-8 week old) where subcutaneously transplanted with a primary HCC tumor fragment. Animals were divided into treatment groups (12 animals in one group) after the tumors were established. Respective groups were administered orally with the 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate at 100mg/kg for 5 out of 7 days. At the end of treatment T/C values were calculated and tumor regrowth was observed.
Results: 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate (100 mg/kg/5 out of 7 days) significantly inhibited the growth of 4 out of the 9 models (LIM612, LIM801, LIM1098, LIM941; T/C values of 49% to -97%). Sorafenib (50 mg/kg/5 out of 7 days) displayed an anti-tumor activity in 7 out of 9 models (LIM348, LIM612, LIM941, LIM752, LIM1098, LIM801, LIM1081 with T/C values of 45% to -8%). 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate exhibited better anti-tumor activity than sorafenib monotherapy in LIM801 and LIM612, two models with strong signs of c-Met signaling. The combination of sorafenib with 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate enhanced anti-tumor activity of best monotherapy in 2 out of 9 models (LIM1098 and LIM752 with T/C values of -32% and 4%, respectively).

### Determination of c-Met phospho c-Met and HGF protein levels in primary explants:

Method: c-Met, phospho c-Met, and HGFalpha expression was studied with IHC in satellite animals of human primary tumor explants and xenografts (Table 1). The xenografts were excised, sectioned into few pieces, fixed in 4% buffered formaldehyde solution during 48 hrs at RT and embedded in paraffin. Sections of 3µm of formaldehyde fixed paraffin embedded (FFPE) tissue were mounted on positively charged SuperFrost®Plus slides (Menzel-Gläser, Braunschweig, Germany). The immunohistochemical staining procedure starting with the deparaffinization of sections was done with the staining instrument Discovery™ or the Discovery® XT (Ventana Medical Systems, Inc., Tucson, USA). After deparaffinization sections were heated for epitope retrieval in Tris-EDTA buffer pH 8. Endogenous peroxidase was blocked by incubation in 3% hydrogen peroxide (part of OmniMap™ Kit, Ventana Medical Systems). Sections were incubated with in PBS diluted antibodies. and then with the secondary antibody, the HRP conjugated polymers of the OmniMap Kit, for 16 min at 37°C. Horseradish peroxidase (HRP) catalyzes the 3,3'-diaminobenzidine tetrahydorchloride (DAB)/H2O2 reaction to produce an insoluble dark brown precipitate that can be visualized. Sections were counterstained with hematoxylin. Slides were washed in tap water, dehydrated, and mounted with glass coverslips in permanent mounting media Entellan® Neu (VWR, Germany). The detailed run protocols, generated by the staining instruments, are stored at Merck Serono, Darmstadt, Germany. Immunohistochemical stainings were scanned with the help of the MiraxSCAN (Zeiss) with a resolution x/y: 1 pixel = 0.23 x 0.23 µm2. The scannings were analyzed with the image analysis software Visiopharm Integrator System (VIS; V 4.0.3.0; Visiopharm A/S, Denmark). Viable tissue area was outlined avoiding obvious necrotic areas and connective tissue. For the determination of the amount of antigen present, positive brown stained area was calculated as percent area of the viable tissue area. Antibody staining (arbitrary units) is calculated as Antibody staining (AU) = Positive area (%) * (255-Intensity)/100 of the brown colour.
Results: In explants of human hepatocellular carcinoma (HCC), high c-Met, phospho-Met and moderate HGFalpha expression could be detected in single explants with the help of immunohistochemistry (IHC). Out of 9 HCC explants, 8 were positive for c-Met. Two of the explants (LIM1098 and LIM612) showed high pTyr 1234/1235 Met and pTyr 1349 Met expression. Additional 3 explant tumors showed low to moderate pTyr1349-Met expression. Due to high background staining with the detection system for the HGF antibody clone B-3 (mouse IgG) in several explant xenografts, the tumors could not be analyzed with the help of image analysis. A semiquantitative scoring was performed (C. Wilm) by comparing the specific anti-HGF staining with the mouse IgG isotype control staining.
Scores are:
0 = negative
1 = low
2 = medium
3 = high

Two out of 9 HCC explants exhibited low to moderate HGF alpha expression. LIM612 with low HGF expression was highly positive for phospho-Met. LIM801 with moderate HGF expression was negative for phospho-Met.

## Claims

1. 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof for the use for the treatment of hepatocellular carcinoma (HCC).

2. 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate for the use for the treatment of hepatocellular carcinoma (HCC).

3. 3-(1-{3-[5-(1-methyl-piperidin-4-ylmethoxy)-pyrimidin-2-yl]-benzyl}-6-oxo-1,6-dihydro-pyridazin-3-yl)-benzonitrile according to claim 1 or 2, wherein the compound is administered to a patient in an amount of 100 mg to 800 mg per day.

4. 3-(1-{3-[5-(1-methyl-piperidin-4-ylmethoxy)-pyrimidin-2-yl]-benzyl}-6-oxo-1,6-dihydro-pyridazin-3-yl)-benzonitrile according to claim 1, 2 or 3, wherein the compound is administered orally.

## Patentansprüche

1. 3-(1-{3-[5-(1-Methyl-piperidin-4-ylmethoxy)-pyrimidin-2-yl]-benzyl}-6-oxo-1,6-dihydro-pyridazin-3-yl)-benzonitril oder ein pharmazeutisch unbedenkliches Salz und/oder Solvat davon zur Verwendung für die Behandlung von hepatozellulärem Karzinom (hepatocellular carcinoma - HCC).

2. 3-(1-{3-[5-(1-Methyl-piperidin-4-ylmethoxy)-pyrimidin-2-yl]-benzyl}-6-oxo-1,6-dihydro-pyridazin-3-yl)-benzonitril-hydrochlorid-hydrat zur Verwendung für die Behandlung von hepatozellulärem Karzinom (HCC).

3. 3-(1-{3-[5-(1-Methyl-piperidin-4-ylmethoxy)-pyrimidin-2-yl]-benzyl}-6-oxo-1,6-dihydro-pyridazin-3-yl)-benzonitril nach Anspruch 1 oder 2, wobei die Verbindung einem Patienten in einer Menge von 100 mg bis 800 mg pro Tag verabreicht wird.

4. 3-(1-{3-[5-(1-Methyl-piperidin-4-ylmethoxy)-pyrimidin-2-yl}-benzyl}-6-oxo-1,6-dihydro-pyridazin-3-yl)-benzonitril nach Anspruch 1, 2 oder 3, wobei die Verbindung oral verabreicht wird.

## Revendications

1. 3-(1-{3-[5-(1-Méthylpipéridin-4-ylméthoxy)pyrimidin-2-yl]benzyl}-6-oxo-1,6-dihydropyridazin-3-yl)benzonitrile ou un sel et/ou solvate pharmaceutiquement acceptable de celui-ci, pour une utilisation dans le traitement du carcinome hépatocellulaire (CHC).

2. Chlorhydrate de 3-(1-{3-[5-(1-méthylpipéridin-4-ylméthoxy)-pyrimidin-2-yl]benzyl}-6-oxo-1,6-dihydropyridazin-3-yl)benzonitrile hydraté, pour une utilisation dans le traitement du carcinome hépatocellulaire (CHC).

3. 3-(1-{3-[5-(1-Méthylpipéridin-4-ylméthoxy)pyrimidin-2-yl]benzyl}-6 oxo-1,6-dihydropyridazin-3-yl)benzonitrile selon la revendication 1 ou 2, où le composé est administré à un patient selon une quantité allant de 100 mg à 800 mg par jour.

4. 3-(1-{3-[5-(1-Méthylpipéridin-4-ylméthoxy)pyrimidin-2-yl]benzyl}-6-oxo-1,6-dihydropyridazin-3-yl)benzonitrile selon la revendication 1, 2 ou 3, où le composé est administré par voie orale.
